# EUROPEAN PATENT SPECIFICATION

(11) **EP 0 516 135 B1**
(45) Date of publication and mention of the grant of the patent: **04.09.1996**
(21) Application number: 92109072.6
(22) Date of filing: 29.05.1992
(51) Int. Cl.: C12N 15/49, C07K 16/10, G01N 33/569, C12Q 1/70, A61K 39/21, C07K 14/16

(54) **Human immunodeficiency virus-related immune preparation**
Gegen das menschliche Immunschwäche-Virus gerichtetes Immunpräparat
Préparation immunitaire liée au virus immunodéficient humain

(30) Priority: 31.05.1991 JP 129224/91
(43) Date of publication of application: 02.12.1992
(73) Proprietor: Juridical Foundation The Chemo-Sero-Therapeutic Research Institute, Kumamoto-shi Kumamoto-ken (JP)
(72) Inventor: Eda, Yasuyuki, Kikuchi-gun, Kumamoto-ken (JP); Shiosaki, Kouichi, Kikuchi-gun, Kumamoto-ken (JP); Osatomi, Kiyoshi, Pana Haitsu 101, Kumamoto-shi, Kumamoto-ken (JP); Tokiyoshi, Sachio, Kumamoto-shi, Kumamoto-ken (JP)
(74) Representative: VOSSIUS & PARTNER

(56) References cited:
- WO-A-89/03391
- WO-A-90/03984
- JOURNAL OF IMMUNOLOGY. vol. 145, no. 7, 1 October 1990, BALTIMORE US pages 2199 - 2206 FUNG ET AL. 'Monoclonal anti-idiotypic antibody mimicking the principal neutralization site in HIV-1 gp 120'

## Description

The present invention relate to an immune preparation useful for prevention, treatment and diagnosis of viral infection, more particularly to an immune preparation comprising specific antibodies or peptides useful for prevention, treatment and diagnosis of infection of human immunodeficiency virus.

Human immunodeficiency virus (hereinafter referred to as "HIV") is a human retrovirus which causes a series of diseases such as acquired immunodeficiency syndrome (hereinafter referred to as "AIDS") and AIDS-related syndrome (hereinafter referred to as "ARC"). HIV now spreads all over the world.

Generally speaking, it is a vaccine comprising an antigen of a viral protective region and a neutralizing antibody recognizing said region that plays an important role in the immunological prevention and treatment of viral infection. However, in case of HIV, both of the vaccine and the neutralizing antibody have not yet been put to practical use. This is because the protective region of HIV (env, gp120, etc. ) is easily mutated due to reverse transcriptase (RT) of HIV which is liable to cause errors, and thereby HIV likely escapes from the protective mechanism of the human immune system and is capable of maintaining infection.

Nowadays, AIDS has been treated with an anti-viral agent such as azidothymidine (AZT). However, the treatment with the anti-viral agent has disadvantages such as adverse side effects and an appearance of a new drug-resistant strain. Accordingly, there is a need to develop a vaccine which protects uninfected individuals from HIV infection and to develop a neutralizing antibody against HIV as a treating agent and a diagnostic agent capable of directly measuring such a neutralizing antibody.

At present, the most reliable and promising protective region of HIV is a Cys-ended region (i.e. a region between two cysteins) corresponding to the amino acid sequence 303-338 of the env protein (gp120) which is called "principal neutralizing determinant" (hereinafter referred to as "PND"), as determined by a neutralization test using a monoclonal antibody or a serum obtained by immunization with a synthetic peptide [K. Javaherians et al., Proc. Natl. Acad. Sci. USA 86, 6768-6772 (1989)]. The amino acid sequence of the PND region was analyzed for 245 viral strains isolated from 133 HIV-infected individuals, and as a result, this region was confirmed to be a hypervariable region and it is suggested that this PND region, which is situated in the V3 region within gp120, possibly plays an important role in the adsorption or invasion of HIV to target cells, like the CD4-binding region which is coded in the other region within the env protein [Gregory J. Larosa et al., Science, 249, 932-935 (1990)]. As a result of analysis of the amino acid sequence of the PND region for the above 245 HIV mutants, Larosa et al. also have found that the secondary structure of the PND region has a structural restraint estimated from the amino acid sequence. That is, they report a common structure of β strand - type II β turn - β strand - α helix.

However, when this region (PND) is used for the diagnosis, treatment or prevention of HIV infection, every kinds of the tested PND regions should be taken into consideration, and hence, it cannot be known as to how many kinds of monoclonal antibody or peptide should be used. In addition, as a matter of fact, it is highly possible that a HIV-infected individual is infected with two or more HIV viral strains, and hence, it will be more complicated to find an effective combination of the PND regions. In this sense, although the antigen of the protective region have already been found, this region is a hypervariable region, and it has not yet been determined as to which kind of the PND region should be used as the region to be recognized by an antibody or as the antigen for a vaccine or a diagnostic agent.

The hypervariable region (PND) of HIV is greatly expected as the peptide for enabling the treatment or prevention of HIV infection. In consideration of the mutation peculiar to HIV, it is unlikely that a single peptide corresponding to only one kind of the PND region (hereinafter referred to as "PND peptide") can effectively be used as a medicament effective for every HIV infection by all kinds of HIV strains. However, it is actually quite difficult to develop a pharmaceutical preparation comprising several tens or more peptides.

Under the circumstances, the present inventors have intensively studied in order to find a combination of the PND peptides which is capable of reacting with any of all HIV mutants and which comprises a minimal number of the PND peptides so that the preventing agent and treating agent for HIV infection can be obtained.

An object of the present invention is to provide a peptide preparation which comprises at least two kinds of PND peptides of HIV gp120.

Another object of the present invention is to provide an antibody preparation which comprises at least two specific neutralizing antibodies which recognize the PND peptide of HIV gp120.

A further object of the present invention is to provide an anti-idiotype antibody preparation which comprises at least two anti-idiotype antibodies which recognize the antigen-binding site of a specific neutralizing antibody that recognizes the PND peptide of HIV gp120.

Still another object of the present invention is to provide a method for determining a type of neutralizing antibody against HIV.

Still a further object of the present invention is to provide a method for determining a type of viral strain of HIV.

These and other objects and the advantages thereof will be apparent to those skilled in the art from the following description.

Fig. 1 is a chart showing the secondary structure of various PND peptides obtained by the Robson's analytical method for secondary structure of protein.

Fig. 2 is a chart showing the theoretical typical patterns of the secondary structure in the first part of the PND peptide and the Group-specific secondary structure of various PND peptides which are classified into Groups of I to VI based on said typical patterns of the secondary structure.

Fig. 3 shows an amino acid sequence of the PND peptide in various isolated strains of HIV.

The present inventors have prepared two monoclonal antibodies which specifically bind to the PND region of HIV-MN strain (also called as "HTLV-111 MN strain"; G. Gurgo et al., Virology 164, p531-536, 1988) and conducted an epitope mapping. As a result, the present inventors have found that the antibody with a lower neutralizing antibody titer recognize a primary structure while the antibody with a higher neutralizing antibody titer recognize a superstructure of more than secondary structure.

The present inventors have determined a binding of the PND peptides with antibodies having a strong neutralizing activity against a variety of the PND peptides and classified the PND peptide in groups. Although this can be more directly done by studying the neutralization reaction of isolated viral strains with antibodies, the present inventors have strictly studied a one-to-one reaction of PND peptide with an antibody since such an isolated viral strain may also contain its another mutant strain. That is, the present inventors have expressed a variety of PND peptides derived from HIV-infected individuals in Japan in expression system using E. coli as a host, and studied a binding activity of this purified protein with a neutralizing antibody. As a result, various PND peptides were classified into two groups, i.e. a group which reacted with the antibody and a group which did not reacted with the antibody. The amino acid sequence of PND of the antibody-reacted group was determined, and as would be expected, a common amino acid sequence could not be found in the primary structure.

using an analytical program for secondary structure based on the Robson's estimation theory [Garnier - Osguthorpe - Robson method (GOR method); J. Mol. Biol., 120, 97-120 (1978)], the present inventors have then analyzed a secondary structure of a population of PND peptides which reacted with the antibody, and as a result, have found a common structure among these PND peptides. That is, the secondary structure of the PND peptides which bound to the neutralizing antibody was completely identical to each other in view of the relationship of the β strand in the first part with the GPGR region. This secondary structure could not be found in any of PND peptides which did not reacted with the antibody.

In addition, the present inventors have also conducted the same analysis using the Robson's program of the amino acid sequence of PND for 245 HIV-infected individuals which have been reported till now, and as a result, have confirmed that a structural pattern of the β strand - GPGR region in the first part of PND peptide can be classified into broadly six groups. The present inventors have also found that PND peptides of three groups include in total as high as about 90 % of the PND amino acid of HIV which has hitherto been reported and PND peptides of five groups include almost 100 %.

The "GPGR region" in the present specification means a region having a sequence of Glycine - Proline - Glycine - - Arginine which is well conserved among each HIV strain and corresponds to the amino acids located between the 15th and 18th amino acid in the Cys-ended region in the PND region. On rare occasions, this region also shows a mutation, and in such a case, the GPGR region means a region which corresponds to this position and shows a homology with GPGR. The "β strand region" in the present specification means a region of an amino acid sequence whose secondary structure is estimated as a series of β strands around the 10th amino acid of the PND region when the secondary structure of the PND region is estimated by the Robson's analytical method for secondary structure.

In order to prove the above-mentioned findings, the present inventors have prepared each one PND peptide from each group and immune sera of guinea pig using these peptides. A neutralization test was conducted for clinically isolated strains of HIV using these sera, and as a result, it was found that the immune serum prepared by using the PND peptide from each group could neutralize the cloned virus of the same group. On the other hand, in case of uncloned clinically isolated strains, each of these strains could not always be neutralized with the serum belonging to the same group but these strains could be neutralized if a combination of these immune sera of different groups was used.

In summary, although PND (which is a main protective antigen of HIV) is a hypervariable region, it has a structural restriction. That is, when the secondary structure of the PND peptide is analyzed with the analytical program based on the Robson's theory, the PND peptides can be classified into broadly six groups based on the number of β strand in the vicinity of the 10th amino acid of PND and the distance between these β strands and the first glutamine in the GPGR region (GPGR). Isolated strains of HIV having PND peptide of each group is neutralized in a group-specific manner by a neutralizing antibody which recognizes a superstructure (more than secondary structure) of the β strand - GPGR region in the first part of a PND peptide, and a PND peptide of each group is capable of inducing the group-specific neutralizing antibody and is also capable of detecting the presence of the neutralizing antibody. Therefore, according to the method for classification of the PND peptide of the present invention, a vaccine, a neutralizing antibody and a diagnostic agent for HIV infection can be developed.

Hereinafter, there are explained the classification of the HIV PND peptide, an antibody preparation and an antigen preparation prepared based on said classification, and a treating agent, a vaccine and a reagent for detection using the same.

### (1) Classification of PND peptide by the estimation of secondary structure:

The "PND region" in the present specification means a Cys-ended sequence comprising about 35 amino acids Nos. 303 to 338 counted from the N terminus of HIV gp120 peptide. Although the numbering of the amino acid sequence in this region may alter to a small degree due to deletion or insertion of nucleotide since HIV shows a high liability of mutation, there exists a Cys-ended region comprising about 35 amino acids in the vicinity thereof and such a Cys-ended region is called PND region.

According to the present invention, the peptide for preparation of a vaccine, a reagent or a neutralizing antibody can be selected by subjecting the PND peptide of HIV whose amino acid sequence has already been determined to the analytical theory for superstructure of peptide established by Robson [GOR method; J. Mol. Biol., 120, 97-120 (1987)] to analyze the pattern thereof and determining the number (usually 3 to 5) of β strand shown by the region of 10th to 15th amino acids [wherein the numbering of the amino acid sequence is calculated in such a way that "Cys" at the N terminus of PND (i.e. Cys-ended region) is No. 1] or the region situated in the vicinity thereof and the distance between said β strand and the GPGR region at the C terminus, thereby determining as to which PND group of the present invention the tested PND peptide belongs to.

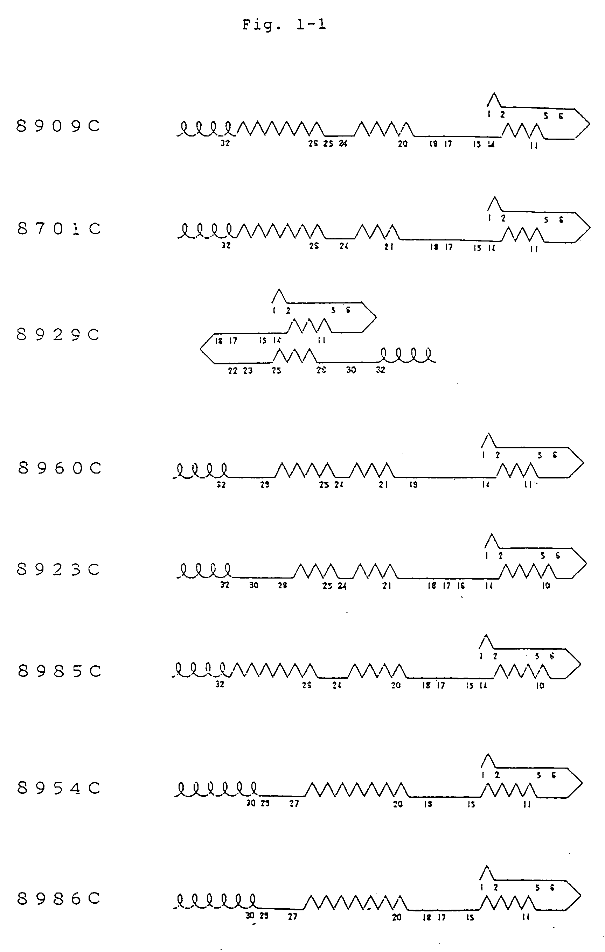

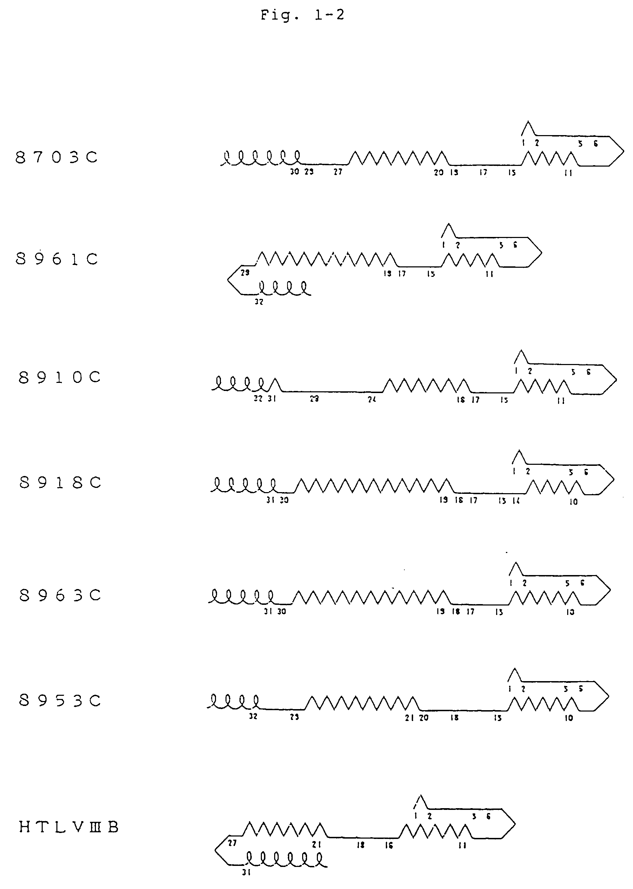

When the secondary structure is estimated from the primary structure according to the Robson's method, either of a helx, β sheet, turn or coil structure is estimated. In the present specification, the "β strand" means the β sheet. In Fig. 1, an upward triangle peak in the vicinity of the 10th amino acid of the PND peptide is referred to a β strand. The analysis of the superstructure from the amino acid sequence according to the Robson's theory can easily be done utilizing commercially available analytical softwares for personal computer (e.g. GENETYX).

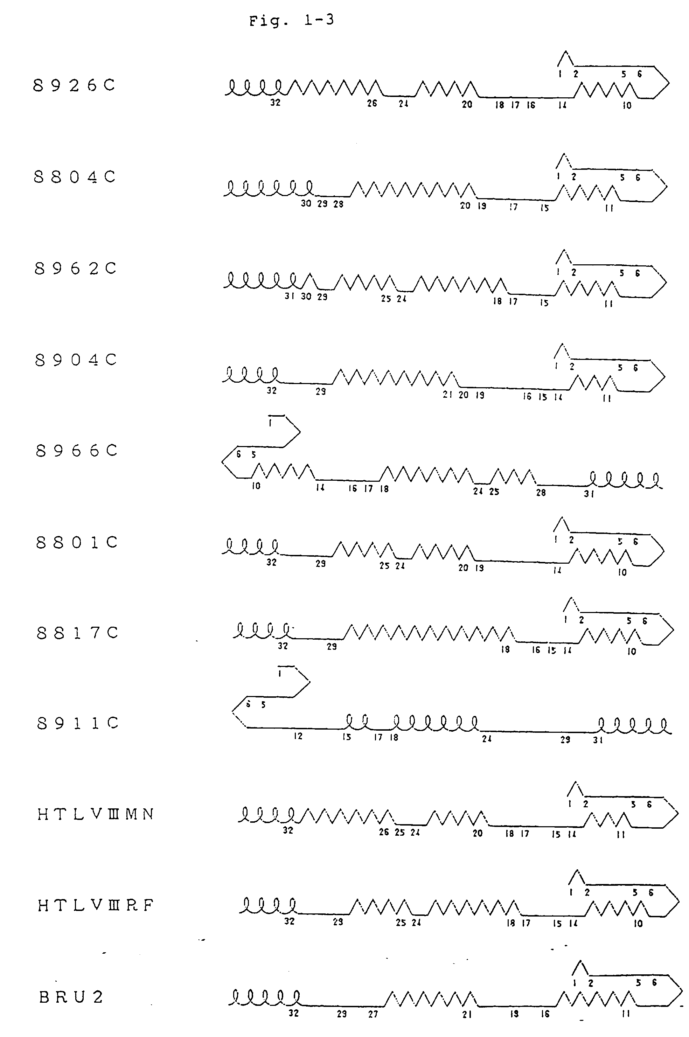

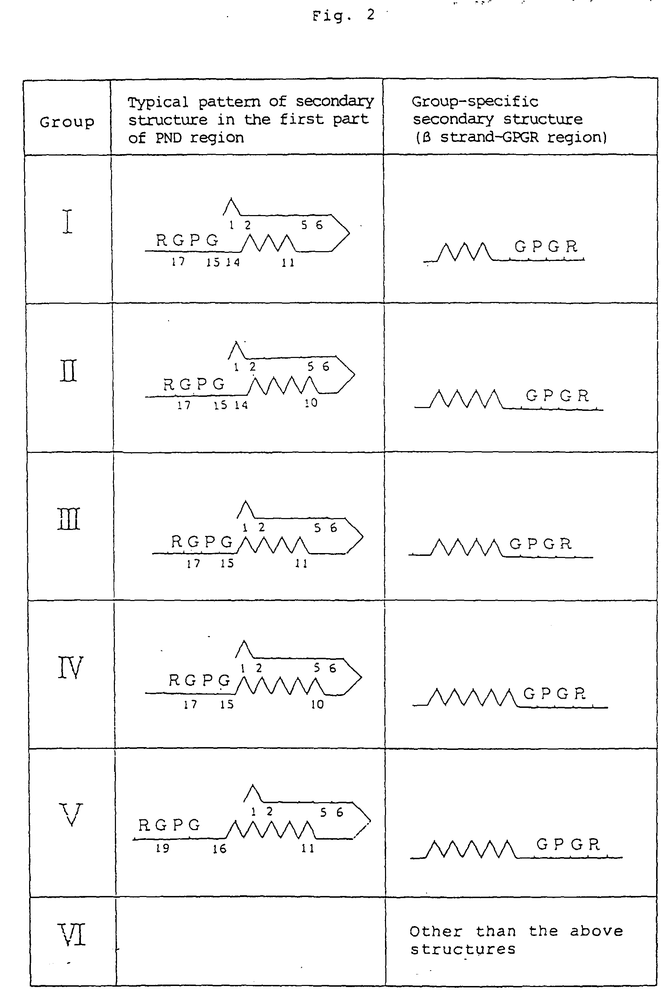

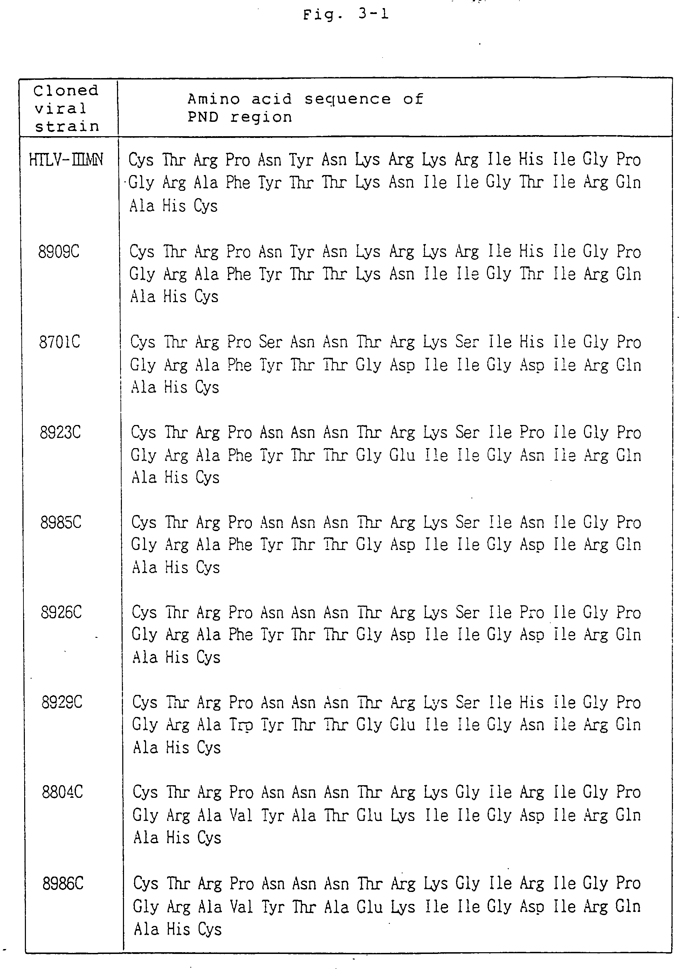

According to the present invention, the PND peptides are classified into five groups (Fig.2), i.e. Group I wherein the number of the β strand is 3, Group II wherein the number of the β strand is 4 and an amino acid having a coily or turn structure is intervened between these β strand structures and the first G of the GPGR region, Group III wherein the number of the β strand is 4 and these β strand structures and the GPGR region are situated adjacent to each other without intervention of other amino acid, Group IV wherein the number of the β strand is 5 and these β strand structures and the GPGR region are situated adjacent to each other without intervention of an amino acid having a coily or turn structure, and Group V wherein the number of the β strand is 5 and an amino acid having a coily or turn structure is intervened between these β strand structures and the GPGR region and Group VI comprising PND peptides having different pattern from those of Groups I to V.

The group-specific patterns of the secondary structure of the PND peptide according to the present invention are summarized using the estimation of the secondary structure of the seven amino acids just, in N-terminal side, adjacent to the GPGR region as follows:
(I) Group I: PND peptide having an amino acid sequence of: (N-terminus) XXXBBBX (C-terminus);
(II) Group II: PND peptide having an amino acid sequence of: XXBBBBX;
(III) Group III: PND peptide having an amino acid sequence of: XXXBBBB;
(IV) Group IV: PND peptide having an amino acid sequence of: XXBBBBB; and
(V) Group V: PND peptide having an amino acid sequence of: XBBBBBX
wherein B represents a β strand structure and X represents a turn structure or a coil structure.

This PND classification of the present invention was used for analysis of the PND amino acid sequence of about 20 HIVs which the present inventors have isolated from HIV-infected individuals and cloned therefrom and 245 PND amino acid sequences which Larosa et al. have reported, and as a result, it was confirmed that about 90 % of all hitherto known HIV strains is included in three groups, i.e. Groups I, II and III, and almost 100 % is included in five groups, i.e. Groups I to V.

### (2) Peptide preparation:

The "peptide preparation" of the present invention means a peptide preparation which comprises at least two PND peptides each belonging to a different Group (either of Groups I to V) of the classification of the present invention. The preferable peptide preparation of the present invention preferably comprises at least three PND peptides each belonging to a different Group (either of Groups I to V), more preferably at least three PND peptides each belonging to a different Group (either of Group I, II or III). Most preferably it comprises at least five PND peptides each belonging to a different Group (either of Groups I to V). The "PND peptide" means a peptide having about 35 amino acids called PND region, or a larger peptide containing the same peptide. There can also be used a smaller peptide which contains the PND region where a part of the N and/or C termini were deleted and has less than 35 amino acids. In this case, the peptide should have a length of at least about 20 amino acids comprising the β strand region and the GPGR region.

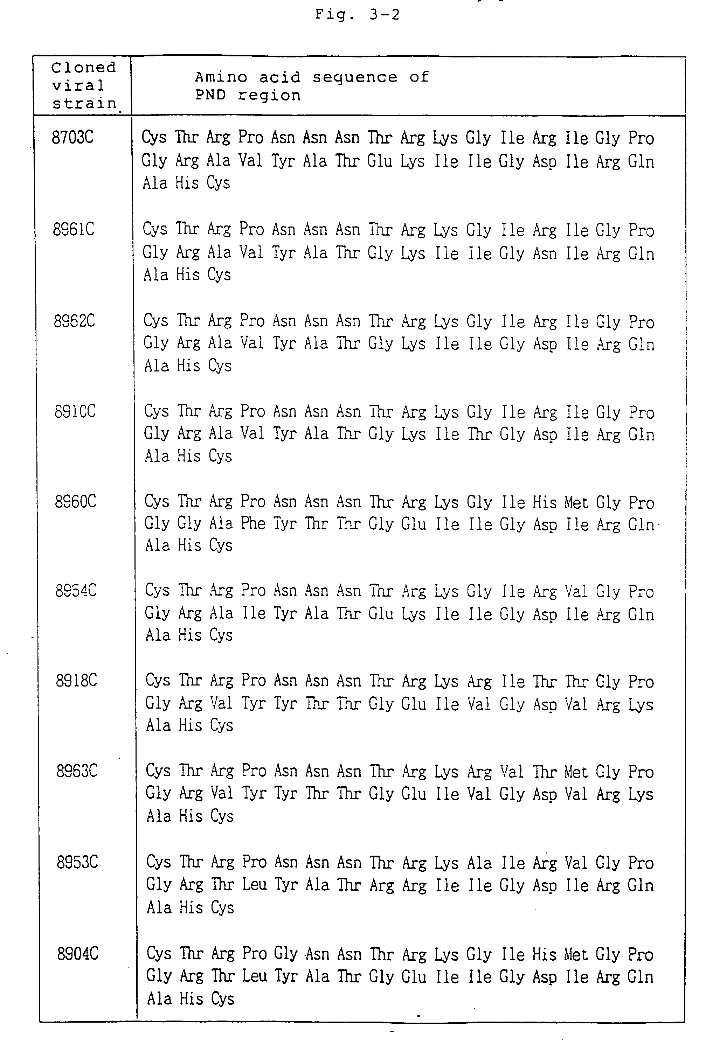

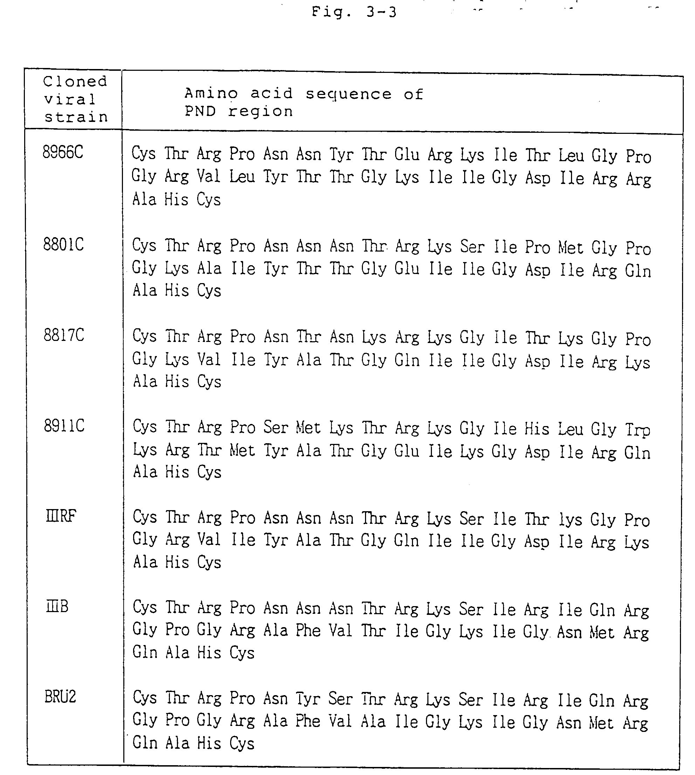

Amino acid sequences of representative PND peptides in each Group are shown in the following. It should be noted that these amino acid sequences merely show the exemplification of PND peptide belonging to each Group but should not be construed to be limited thereto. Fig. 3 shows the same amino acid sequences by a three-letter symbol.

### Group I:

(ⅢMN)    CTRPNYNKRKRIHIGPGRAFYTTKNIIGTIRQAHC

(8909C)    CTRPNYNKRKRIHIGPGRAFYTTKNIIGTIRQAHC

(8701C)    CTRPSNNTRKSIHIGPGRAFYTTGDIIGDIRQAHC

(8929C)    CTRPNNNTRKSIHIGPGRAWYTTGEIIGNIRQAHC

(8960C)    CTRPNNNTRKGIHMGPGGAFYTTGEIIGDIRQAHC

(8904C)    CTRPGNNTRKGIHMGPGRTLYATGEIIGDIRQAHC

### Group II:

(8923C)    CTRPNNNTRKSIPIGPGRAFYTTGEIIGNIRQAHC
(8985C)    CTRPNNNTRKSINIGPGRAFYTTGDIIGDIRQAHC
(ⅢRF)    CTRPNNNTRKSITKGPGRVIYATGQIIGDIRKAHC
(8926C)    CTRPNNNTRKSIPIGPGRAFYTTGDIIGDIRQAHC
(8918C)    CTRPNNNTRKRITTGPGRVYYTTGEIVGDVRKAHC
(8966C)    CTRPNNYTERKITLGPGRVLYTTGKIIGDIRRAHC
(8817C)    CTRPNTNKRKGITKGPGKVIYATGQIIGDIRKAHC
(8801C)    CTRPNNNTRKSIPMGPGKAIYTTGEIIGDIRQAHC

### Group III:

(8804C)    CTRPNNNTRKGIRIGPGRAVYATEKIIGDIRQAHC

(8986C)    CTRPNNNTRKGIRIGPGRAVYTAEKIIGDIRQAHC

(8703C)    CTRPNNNTRKGIRIGPGRAVYATEKIIGDIRQAHC

(8961C)    CTRPNNNTRKGIRIGPGRAVYATGKIIGNIRQAHC

(8962C)    CTRPNNNTRKGIRIGPGRAVYATGKIIGDIRQAHC

(8910C)    CTRPNNNTRKGIRIGPGRAVYATGKITGDIRQAHC

(8954C)    CTRPNNNTRKGIRVGPGRAIYATEKIIGDIRQAHC

### Group IV:

(8963C)    CTRPNNNTRKRVTMGPGRVYYTTGEIVGDVRKAHC

(8953C)    CTRPNNNTRKAIRVGPGRTLYATRRIIGDIRQAHC

### Group V:

(IIIB)    CTRPNNNTRKSIRIQRGPGRAFVTIGKIGNMRQAHC
(BRU2)    CTRPNYSTRKSIRIQRGPGRAFVAIGKIGNMRQAHC

The above peptides can be chemically synthesized using a peptide synthesizer. Alternatively, the genetic engineering technique can also be used so that a fused peptide containing a desired PND peptide is expressed in a suitable host cell and the expressed peptide is collected and purified. These PND peptides used for the peptide preparation of the present invention can also be used in such a way that two or more or all of these peptides are fused into one body.

The peptide preparation of the present invention can be used as an active ingredient of a vaccine for inducing a neutralizing antibody which prevents HIV infection or a vaccine for inducing a neutralizing antibody which prevents outbreak of AIDS. Such a vaccine can be prepared according to the conventional method for preparing a vaccine, for example, by adding an adjuvant if necessary.

### (3) Antibody preparation:

The "antibody preparation" of the present invention means a preparation comprising neutralizing antibodies, each of which specifically recognizes either of PND peptides each belonging to a different Group (either of Groups I to V) according to the classification of the present invention (cf. Fig. 2). That is, the antibody preparation of the present invention is a mixture comprising at least two neutralizing antibodies each belonging to a different Group, i.e. either of the following Groups I' to V' of a neutralizing antibody which specifically recognizes the PND peptide belonging to Groups I to V, respectively.
(I') Group I': neutralizing antibody which specifically recognizes the PND peptide belonging to Group I;
(II') Group II': neutralizing antibody which specifically recognizes the PND peptide belonging to Group II;
(III') Group III': neutralizing antibody which specifically recognizes the PND peptide belonging to Group III;
(IV') Group IV': neutralizing antibody which specifically recognizes the PND peptide belonging to Group IV; and
(V') Group V': neutralizing antibody which specifically recognizes the PND peptide belonging to Group V.

The antibody preparation of the present invention preferably comprises at least three neutralizing antibodies, each of which belongs to a different Group (either of Groups I' to V') and specifically recognizes the PND peptides each belonging to a different Group (either of Groups I to V). More preferably, the antibody preparation of the invention comprises five neutralizing antibodies, each of which belongs to a different Group (either of Groups I' to V') and specifically recognizes the PND peptides each belonging to a different Group (either of Groups I to V). The neutralizing antibody used in the antibody preparation of the invention includes not only a neutralizing antibody which is specific to the primary amino acid sequence of the PND peptide but also a neutralizing antibody which specifically recognizes the superstructure of more than secondary structure of the PND peptide characteristic to each Group (I to V).

The neutralizing antibody used in the antibody preparation of the invention may be a serum obtained from various animals by immunizing them with the above PND peptide, or a monoclonal antibody which is prepared by immunizing mouse with the above PND peptide in accordance with the conventional method for preparing a monoclonal antibody. The neutralizing antibody can be prepared by immunizing animals with a peptide belonging to one of Groups I to V to produce a neutralizing antibody which specifically recognizes said peptide. The thus prepared Group-specific neutralizing antibodies each belonging to a different Group (either of Groups I' to V') are then mixed together to give the antibody preparation of the present invention. From the obtained antibodies, those antibodies having a high neutralizing activity which recognize the superstructure of more than secondary structure of PND characteristic to the groups classified according to the present invention can be screened by selecting an antibody which specifically reacts with both the immunized peptide and other PND peptide which belongs to the same Group as the immunized peptide but has a different primary amino acid sequence from the immunized peptide.

For use in human beings as a preventive or treating agent, the antibody preparation of the invention preferably comprises a human-type monoclonal antibody, secreted by a mouse x human hybridoma and a human x human hybridoma. Utilizing the genetic engineering technique, there can also be used a chimeric antibody recognizing the desired secondary structure which is prepared by linking the variable region of a mouse x mouse monoclonal antibody to the constant region of a human antibody, or a human reshaped neutralizing monoclonal antibody which is prepared by replacing only the complementarity determining regions (CDRs) of the variable region of human IgG with the CDRs from mouse neutralizing monoclonal antibodies, as described by Winter (GB patent publication No. 2,188,638, priority: March 27, 1986). These specific chimeric antibodies can be prepared by the known method, for example, as described in European patent publication No. 327000A.

### (4) Anti-idiotype antibody preparation:

The "anti-idiotype antibody" in the present invention means an antibody specific to the antigen-binding site (idiotype) of the neutralizing antibody which specifically recognizes the PND peptide belonging to any of Groups I to V as classified by the present invention (cf. Fig. 2). These anti-idiotype antibody can induce a neutralizing antibody which prevents HIV infection by immunizing human or various animals.

The "anti-idiotype antibody preparation" of the present invention means a preparation comprising at least two anti-idiotype antibodies each specific to the neutralizing antibodies, each of which specifically recognizes the PND peptides each belonging to a different Group (either of Groups I to V ) as classified by the present invention (cf. Fig. 2). That is, the anti-idiotype antibody preparation of the invention means a mixture comprising at least two anti-idiotype antibodies against the neutralizing antibody which specifically recognizes the PND peptide belonging to either of Groups I to V, wherein each anti-idiotype antibody belongs to a different Group (either of the following Groups I'' to V''):
(I'') Group I'': anti-idiotype antibody against the neutralizing antibody which specifically recognizes the PND peptide belonging to Group I;
(II'') Group II'': anti-idiotype antibody against the neutralizing antibody which specifically recognizes the PND peptide belonging to Group II;
(III'') Group III'': anti-idiotype antibody against the neutralizing antibody which specifically recognizes the PND peptide belonging to Group III;
(IV'') Group IV'': anti-idiotype antibody against the neutralizing antibody which specifically recognizes the PND peptide belonging to Group IV; and
(V'') Group V'': anti-idiotype antibody against the neutralizing antibody which specifically recognizes the PND peptide belonging to Group V.

The anti-idiotype antibody preparation of the invention preferably comprises three anti-idiotype antibodies each specific to the neutralizing antibodies, each of which specifically recognize the PND peptides each belonging to a different Group (either of Groups I to V). More preferably, the anti-idiotype antibody preparation of the invention comprises five anti-idiotype antibodies each specific to the neutralizing antibodies, each of which specifically recognize the PND peptides each belonging to a different Group (either of Groups I to V).

The anti-idiotype antibody used in the anti-idiotype antibody preparation of the invention may be a serum obtained from various animals by immunizing them with the above neutralizing antibody, or a monoclonal antibody which is prepared by immunizing mouse with the neutralizing antibody derived from mouse in accordance with the conventional method for preparing a monoclonal antibody. The anti-idiotype antibody can be prepared by immunizing animals with a neutralizing antibody belonging to one of Groups I' to V' to produce an anti-idiotype antibody which specifically recognizes said neutralizing antibody. The thus prepared Group-specific anti-idiotype antibodies each belonging to a different Group (either of Groups I'' to V'') are then mixed together to give the anti-idiotype antibody preparation of the present invention.

Like the antibody preparation as mentioned above, for use in human beings as a preventing or treating agent, the anti-idiotype antibody preparation of the invention preferably comprises a human-type monoclonal antibody, secreted by a mouse x human hybridoma and a human x human hybridoma. Utilizing the genetic engineering technique, there can also be used a chimeric antibody which is prepared by linking the variable region of a mouse x mouse monoclonal antibody to the constant region of a human antibody, or a human reshaped neutralizing monoclonal antibody which is prepared by replacing only the complementarity determining regions (CDRs) of the variable region of human IgG with the CDRs from mouse neutralizing monoclonal antibodies, as described by Winter (GB patent publication No. 2,188,638, priority: March 27, 1986). These specific chimeric antibodies can be prepared by the known methods as mentioned above for the antibody preparation.

The anti-idiotype antibody preparation of the present invention can be used as an active ingredient of a vaccine for inducing a neutralizing antibody which prevents HIV infection or a vaccine for inducing a neutralizing antibody which prevents outbreak of AIDS. Such a vaccine can be prepared according to the conventional method for preparing a vaccine, for example, by adding an adjuvant if necessary.

### (5) Determination of the type of human immunodeficiency virus (HIV):

The present invention provides a method for determining a type of human immunodeficiency virus (HIV) based on the classification of the PND peptide of the present invention. Identification of HIV strain isolated from HIV-infected individuals can be a quite important diagnosis which can be effectively utilized for the treatment of AIDS by administering the antibody specific to said HIV strain. That is, the identification of HIV strain isolated from an HIV-infected individual makes it possible to use the most effective neutralizing antibody which specifically recognizes said HIV strain.

HIV strain can be identified by extracting an HIV DNA genome or a part thereof from blood of HIV-infected individual, incorporating the HIV DNA fragment into E. coli plasmid to clone the same, determining a nucleotide sequence of DNA coding for the PND region of HIV env gp120, and subjecting the primary amino acid sequence of the PND deduced from said nucleotide sequence to the Robson's analytical method for amino acid secondary structure (GOR method), thereby determining as to which Group (Groups I to. V) of PND peptide as classified by the present invention said HIV viral strain belongs to.

### (6) Application to diagnostic agent:

As explained hereinabove, there is a close relationship between the secondary structure of the PND peptide and the neutralizing activity of the antibody. The detection of the presence of HIV having the Group-specific PND or the Group-specific antibody based on the grouping of the secondary structure according to the present invention will be an effective means to estimate the outbreak of AIDS in HIV-infected individuals in future. Accordingly, the present invention provides an assay kit for measuring an antibody which recognizes the Group-specific gp120 antigen or the Group-specific PND peptide based on the grouping of the PND peptide of the present invention. Such an assay kit for determination of antigen/antibody mav be any type found in the conventional assay kit for determination.

The present inventors have first disclosed the relationship between the secondary structure of the PND peptide and the HIV neutralization and found that the PND peptide can be classified into groups based on the Group-specific neutralizing antibodies. The peptide preparation or the antibody preparation of the present invention effective for prevention or treatment of infection of various HIV mutant strains can be obtained by using at least two peptides or neutralizing antibodies each belonging to a different Group (either of Groups I to V ) according to the classification of the invention. Particularly, the use of three peptides or neutralizing antibodies each belonging to a different Group (either of Group I, II or III) provides an immune preparation which can effectively prevent or treat 90 % of any HIV infections. In addition, the use of five peptides or neutralizing antibodies each belonging to a different Group (either of Group I, II, III, IV or V) provides an immune preparation which can effectively prevent or treat almost 100 % of any HIV infections. That is, the classification of the PND peptide allows for development of vaccine, neutralizing antibody and diagnosing agent actually useful for HIV infection.

The present invention is explained in more detail by the following Examples but should not be construed to be limited thereto.

### Example 1

### (1)Expression of HIV PND peptide in E. coli:

HIV-infected peripheral blood mononucleic cell (PBMNC) was isolated from blood of HIV-infected individual which showed positive RT activity using a lymphocyte separating solution. A DNA fragment coding for PND peptide of the HIV-infected individual was amplified by the polymerase chain reaction (PCR) using the genome DNA isolated and purified from PBMNC as a template. The amplification primers used for the PCR were: 5' primer: (5') 6540-GGATCCACACATGGAATTAGGCCAGTA-6562 (3') and 3' primer: (3') 6892-AGTCCTCCCCTGGGTCTTTAAACTGACGTC-6913 (5') wherein the numerals at both ends are the nucleotide Nos. showing the position of the primers in the HIV genome (L. Ratner et al., Nature 313, 277, 1985).

The amplified DNA fragments were digested with the restriction enzymes BamHI and PstI and the digested fragments were inserted into the same restriction enzyme sites in pUEX1 plasmid (Amersham). An E. coli transformant containing the plasmid (a host cell is E. coli HB101 strain; commercially available from Takara Shuzo K.K., Japan) was cultured in L culture medium (10 ml) for 2 hours. The culture temperature was then shifted from 37°C to 42°C and the culture was continued for additional 2 hours to produce the desired PND peptide in a form of a fused protein with β-galactosidase.

### (2) Determination of the amino acid sequence of PND peptide of HIV:

The E. coli transformant prepared in the process (1) was suspended in L culture medium (10 ml) and cultured at 37°C for 12 hours. The cells were collected by centrifugation and the plasmid DNA was isolated by the alkali method. The obtained plasmid was alkali-denatured, annealed with a sequencing primer: (5') 6569-CAACTCAACTGCTGTTAAATGG-6589 (3') and then the nucleotide sequence of the plasmid DNA was determined by dideoxy method.

### (3) Purification of the expressed PND protein:

The PND fused protein was purified by the following procedure. The cells were collected from the culture where the temperature induction was completed. .To the cells were added 50 mM phosphate buffer (pH 7.5; 1 ml) and glass beads (1 ml) and the mixture was vigorously shaked to destroy the E. coli cells. The cells were washed twice with phosphate buffer containing 10 mM EDTA and suspended in phosphate buffer containing 1 mg/ml of lysozyme for about 2 hours to proceed the enzyme digestion. The cells were washed twice with phosphate buffer containing 0.5 % Triton X-100 and then with phosphate buffer and solubilized with 8 M urea. Unsolubilized fractions were removed by centrifugation and the supernatent was collected as a purified fraction. A protein level of this fraction was determined with Bradford kit for protein manufactured by Bio-Rad.

### (4) Binding activity of expressed PND protein with neutralizing monoclonal antibody:

The purified antigen obtained in the process (3) was subjected to a 2-fold continuous dilution with the initial concentration of about 1000-fold dilution (2 µg/ml) and coated onto a 96-well plate (Maxsoap; manufactured by Nunc) at 100 µl/well. The plate was blocked with 2 % BSA solution and thereto was added 4 µg/ml of neutralizing antibodies (µ5.5; FERM BP-3402, Japanese Patent Application No. 188300/1990) and the plate was incubated at 37°C for 2 hours. The plate was washed three times with 0.1 % Tween 20/PBS and thereto was added a solution of a peroxidase-labelled anti-mouse immunoglobulin antibody (manufactured by Cappel; 5,000-fold dilution) at 100 µl/well. The plate was incubated at 37°C for 1 hour and then washed 5 times with 0.1 % Tween 20/PBS. A TMBZ substrate solution was added and the coloring reaction was conducted in the conventional manner and the absorbance was measured at the wave length of 450 nm.

The reactivity of the expressed PND protein with the neutralizing antibody was determined as mentioned above. As a result, it was found that the expressed PND protein derived from HIV-MN strain was reacted strongly with the neutralizing monoclonal antibody against HIV-MN strain (µ5.5) while this neutralizing monoclonal antibody was not reacted with the expressed PND proteins derived from HIV-IIIB=H9/HTLV-IIIB (ATCC No. CRL8543) and HIV-RF. Conversely, the neutralizing monoclonal antibody against HIV-IIIB strain (0.5β) was reacted with the expressed PND protein derived from HIV-IIIB strain but not reacted with the expressed PND protein derived from HIV-MN strain. These results suggest the specificity of the detection system of the invention. Using the similar procedure, there was determined the binding activity of the neutralizing monoclonal antibody against HIV-MN strain with expressed PND proteins from HIV-infected individuals. The results are shown in Table 1.

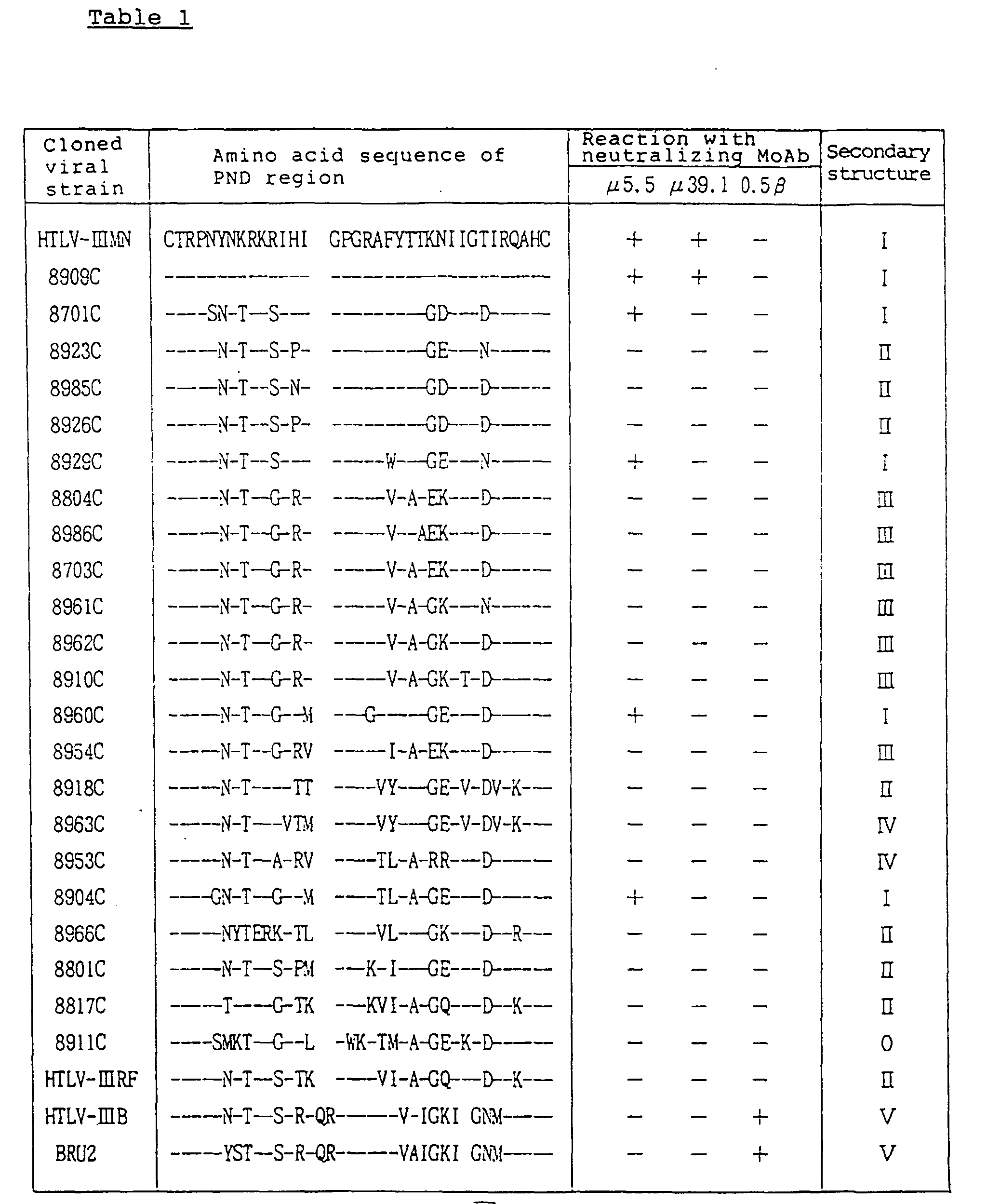

### (5) Analysis of secondary structure of expressed PND protein:

The expressed PND proteins from HIV-infected individuals were divided into groups based on the reactivity with the neutralizing monoclonal antibody and the amino acid sequence of each PND protein was determined. The obtained amino acid sequence was processed with a Robson's analytical program for secondary structure of protein [commercially available software for personal computer; GENETYX Ver. 7,; parameters have been set up at the time of shipment], which method is generally known as the Garnier - Osguthorpe - Robson method (GOR method); J. Mol. Biol. 120, 97-120 (1978). As a result, it was found that the PND proteins reactive with the neutralizing antibody did not had a common primary amino acid sequence but had a common secondary structure. This common secondary structure was found in a region of β strands (β strands in the first part of PND) - GPGR. It was suggested that the antibody capable of recognizing the superstructure of more than secondary structure of this region is possibly an antibody capable of Group-specifically neutralizing HIV. The results are shown in Fig. 1 and Fig.2. The amino acid sequence of the PND proteins which hitherto have been reported could be classified into six groups based on the number of the β strand and the distance between the β strands and the GPGR sequence. It was also found that about 90 % of all the amino acid sequence of the PND proteins was included in three main groups, i.e. Group I, II and III. The results are shown in Table 2.

**Table 2**

| | Group classified based on the secondary structure | | | | | |
|---|---|---|---|---|---|---|
| | I | II | III | IV | V | VI (the others) |
| No. of HIV clone (%) | 86 (32.5%) | 100 (37.7%) | 38 (14.3%) | 17 (6.4%) | 18 (6.8%) | 6 (2.3%) |

### Example 2

### (1) Immunization of guinea pig with synthetic peptide:

Total 13 PND peptides belonging to either of Groups I to V were chemically synthesized using ABI430A peptide synthesizer (manufactured by Applied Biosystem). The amino acid sequences of these peptides are shown in Table 3. These synthesized peptides were bound to keyhole limpet hemocyanin (KLH) to prepare a peptide - KLH conjugate. Guinea pig was immunized with the conjugate together with Freund's complete adjuvant (initial immunization) or with Freund's incomplete adjuvant (secondary immunization) in an amount of 100 µg/dose at an interval of two weeks. Two weeks after the secondary immunization, the animal was further immunized twice with the conjugate without the adjuvant at an interval of one day. These immunizations were all conducted by the intradermal administration. A week after the final immunization, serum was harvested from the animal. The obtained serum was then partially purified with ammonium sulfate and used in the following neutralization test.

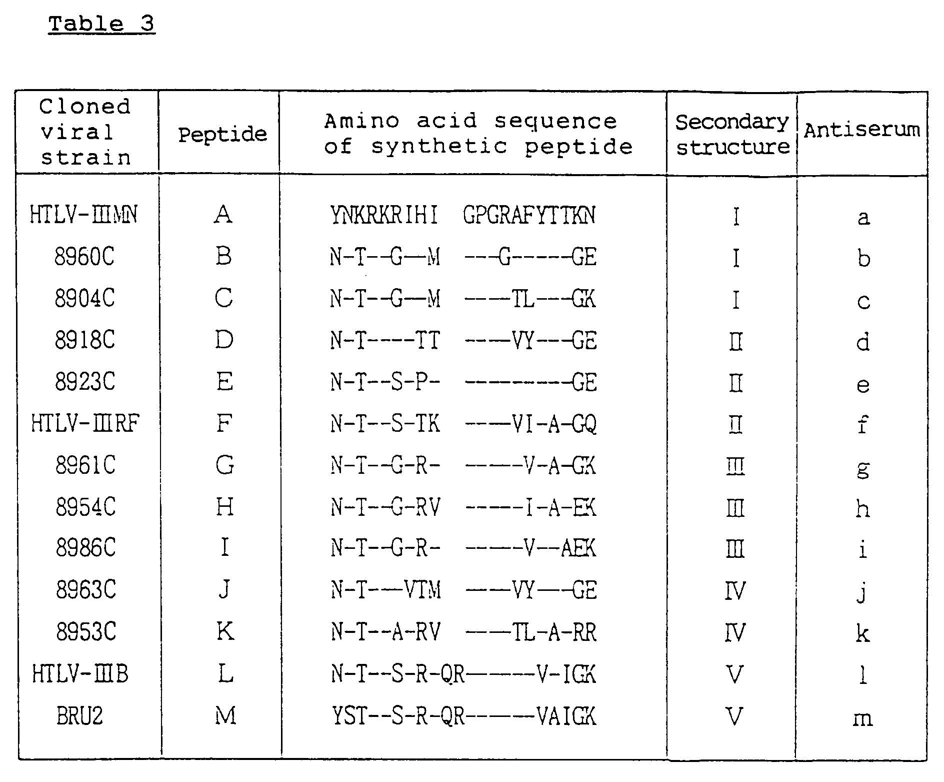

### (2) Binding test of serum obtained by immunization of guinea pig with various synthetic PND peptides:

Using the EIA method described in Example 1 (3), the binding activity of each partially purified guinea pig serum with various synthetic PND peptides shown in Table 3 was determined. The results are shown in Table 4. The binding activity was shown as the multiple dilution of each serum which showed OD₂₈₀ of 0.5 in EIA. Each of the sera in one group was reacted equally and highly with every peptide in the same group, and hence, it was confirmed that sufficiently Group-specific antibodies were contained in each serum.

**Table 4**

| | Peptide | I | | |
|---|---|---|---|---|
| Serum | | A | B | C |
| I | a | 24,000 | 10,000 | 12,000 |
| | b | 8,500 | 12,000 | 7,500 |
| | c | 10,000 | 18,000 | 21,000 |

| | Peptide | II | | |
|---|---|---|---|---|
| Serum | | D | E | F |
| II | d | 12,000 | 7,500 | 25,000 |
| | e | 32,000 | 61,000 | 12,000 |
| | f | 25,000 | 12,000 | 48,000 |

| | Peptide | III | | |
|---|---|---|---|---|
| Serum | | G | H | I |
| III | g | 28,000 | 15,000 | 9,000 |
| | h | 4,800 | 10,000 | 7,500 |
| | i | 3,800 | 9,500 | 12,000 |

| | Peptide | IV | | |
|---|---|---|---|---|
| Serum | | J | K | |
| IV | j | 28,000 | 15,000 | |
| | k | 4,800 | 10,000 | |

| | Peptide | V | | |
|---|---|---|---|---|
| Serum | | L | M | |
| V | l | 16,000 | 16,000 | |
| | m | 20,000 | 24,000 | |

### (3) Neutralization test using the sera obtained by immunization of guinea pig:

There were used viruses isolated from patients having each PND amino acid sequence and cloned viruses thereof (10⁴ - 10⁶ TCID₅₀/ml) in the neutralization test. The cloning of virus was conducted by the limiting dilution method or the plaque method on CD4-Hela cells. For the above isolated viruses and the cloned viruses thereof, the PND amino acid sequence was again confirmed using the PCR-sequencing method as in Example 1 (2) (shown in Table 3).

The isolated viruses and the cloned viruses thereof were prepared to 10 TCID₅₀/50 µl. The obtained viruses were inoculated onto a flat-bottomed 96-well culture plate together with 50 µl of the partially purified immunization serum which was stepwise diluted at 10-fold and the plate was incubated at 37°C for 1 hour. To the plate were added MT4 cells at 10⁴ cells/100 µl/well (cells were floated in RPMI 1640 culture medium containing 10% FCS, 3.5 - 4.0 g/l of L-glutamine, 50 U/ml of penicillin and 50 µg/ml of streptomycin) and cultured for 5 to 6 days. The neutralizing activity of the antibody was determined by as to whether or not the syncytium formation induced by the infection was inhibited by the antibody. The neutralizing activity was shown as the highest dilution which could inhibit the syncytium formation by the cell-free viral infection by 100 %. The results are shown in Table 5.

**Table 5**

| Cloned viral strain | | I | | | II | | | III | | | IV | | V | |
|---|---|---|---|---|---|---|---|---|---|---|---|---|---|---|
| Serum | | IIIMN | 8960C | 8904C | 8918C | 8923C | IIIRF | 8961C | 8954C | 8986C | 8963C | 8953C | IIIB | BRU2 |
| I | a | 256 | 128 | 64 | - | - | - | - | - | - | - | - | - | - |
| | b | 64 | 128 | 64 | - | - | - | - | - | - | - | - | - | - |
| | c | 128 | 256 | 256 | - | - | - | - | - | - | - | - | - | - |
| II | d | - | - | - | 128 | 64 | 128 | - | - | - | - | - | - | - |
| | e | - | - | - | 256 | 512 | 128 | - | - | - | - | - | - | - |
| | f | - | - | - | 256 | 128 | 512 | - | - | - | - | - | - | - |
| III | g | - | - | - | - | - | - | 256 | 128 | 64 | - | - | - | - |
| | h | - | - | - | - | - | - | 32 | 128 | 64 | - | - | - | - |
| | i | - | - | - | - | - | - | 32 | 64 | 128 | - | - | - | - |
| IV | j | - | - | - | - | - | - | - | - | - | 64 | 32 | - | - |
| | k | - | - | - | - | - | - | - | - | - | 32 | 128 | - | - |
| V | l | - | - | - | - | - | - | - | - | - | - | - | 256 | 256 |
| | m | - | - | - | - | - | - | - | - | - | - | - | 256 | 512 |
| I | µ5.5 | 1024 | 256 | 512 | - | - | - | - | - | - | - | - | - | - |
| V | 0.5 β | - | - | - | - | - | - | - | - | - | - | - | 1024 | 1024 |
| (Note) -: No neutralizing activity | | | | | | | | | | | | | | |

The sera in Group I specifically could neutralize any of the cloned viruses in Group I but could not neutralize the cloned viruses in other Groups. Likewise, the sera in each Group II, III, IV and V specifically neutralized the cloned viruses in the same Group as the sera but could not neutralize the cloned viruses in the different Groups from the sera. Each of the sera in one Group could equally neutralize every cloned virus in the same Group. The same results were obtained when the neutralizing monoclonal antibodies belonging to Group I (µ5.5) and Group V (0.5β) were used. The above results proved that the grouping of HIV serum (six grouping) as shown in Example 1 (5) was correct based on the neutralizing activity.

The same neutralization test was also conducted for uncloned isolated viruses. The results are shown in Table 6 wherein the neutralizing activity was shown as the highest dilution of serum which could inhibit the syncytium formation by the cell-free viral infection by 100 %. Each of the sera in one Group could neutralize most of the isolated viruses in the same Group but could not neutralize every isolated viruses in the same Group. This was supposed to be due to the presence of additional virus belonging to other Groups which cannot be neutralized by a single serum.

**Table 6**

| Uncloned viral strain | | I | | | II | | | III | | | IV | | V | |
|---|---|---|---|---|---|---|---|---|---|---|---|---|---|---|
| Serum | | IIIMN | 8960 | 8904 | 8918 | 8923 | IIIRF | 8961 | 8954 | 8986 | 8963 | 8953 | IIIB | BRU2 |
| I | a | 128 | 128 | - | - | - | - | - | - | - | - | - | - | - |
| | b | 64 | 64 | - | - | - | - | - | - | - | - | - | - | - |
| | c | 128 | 128 | - | - | - | - | - | - | - | - | - | - | - |
| II | d | - | - | - | - | 64 | 128 | - | - | - | - | - | - | - |
| | e | - | - | - | - | 512 | 128 | - | - | - | - | - | - | - |
| | f | - | - | - | - | 64 | 512 | - | - | - | - | - | - | - |
| III | g | - | - | - | - | - | - | - | 128 | - | - | - | - | - |
| | h | - | - | - | - | - | - | - | 64 | - | - | - | - | - |
| | i | - | - | - | - | - | - | - | 64 | - | - | - | - | - |
| IV | j | - | - | - | - | - | - | - | - | - | 64 | - | - | - |
| | k | - | - | - | - | - | - | - | - | - | 64 | - | - | - |
| V | l | - | - | - | - | - | - | - | - | - | - | - | 256 | 256 |
| | m | - | - | - | - | - | - | - | - | - | - | - | 256 | 512 |
| I | µ5.5 | 1024 | 512 | - | - | - | - | - | - | - | - | - | - | - |
| V | 0.5 β | - | - | - | - | - | - | - | - | - | - | - | 1024 | 1024 |
| (Note) -: No neutralizing activity | | | | | | | | | | | | | | |

In order to prove the above result, the neutralization test was conducted or the same uncloned isolated viruses using a mixture of five sera each belonging to a different Group (a + f + h + k + l). The results are shown in Table 7 wherein the neutralizing activity was shown as the highest dilution of serum which showed 100 % inhibition of syncytium formation by the cellfree viral infection. A mixture of five sera could neutralize uncloned virus which could not be neutralized by a single serum as shown in Table 6. This proved that a PND peptide preparation comprising five PND peptides each belonging to a different Group (I, II, III, IV and V) can induce a mixture of antibodies which is capable of preventing every type of HIV infections, and hence, is effective as a vaccine for HIV infection.

**Table 7**

| Uncloned viral strain | I | | | II | | | III | | | IV | | V | |
|---|---|---|---|---|---|---|---|---|---|---|---|---|---|
| Serum | IIIMN | 8960 | 8904 | 8918 | 8923 | IIIRF | 8961 | 8954 | 8986 | 8963 | 8953 | IIIB | BRU2 |
| A mixture of five sera | 64 | 32 | 16 | 16 | 16 | 64 | 64 | 32 | 16 | 16 | 16 | 64 | 64 |

### Example 3 (Preparation of a mixture of neutralizing monoclonal antibodies)

### (1) Preparation of each Group-specific neutralizing monoclonal antibody:

The present inventors have already found that the monoclonal antibody 0.5β (Japanese Patent Application No. 131226/1988) is a neutralizing monoclonal antibody specific to Group V. The present inventors have also established the monoclonal antibody µ5.5 as a neutralizing monoclonal antibody specific to Group I (Japanese Patent Application No. 188300/ 1990).

For illustrating the preparation of the neutralizing monoclonal antibodies specific to each Group, the preparation of the monoclonal antibody µ5.5 is described, as follows.

BALB/c mice were immunized with each 100 µg of a conjugated antigen comprising a synthetic peptide [YNKRKRIHIGPGRAFYTTKN(C)] corresponding to the amino acid Nos. 303-325 in the coat glycoprotein gp120 of HTLV-IIIMN strain (belonging to Group I) and keyhole lympet hemocyanin (KLH) together with Freund's complete adjuvant. The immunization was conducted by intraperitoneal injections (three times) and then intravenous injection. Three days after the final immunization, spleen was removed and subjected to cell fusion with the mouse myeloma cell P3x63Ag8-U1 in the conventional manner.

The hybridomas were selectively cultured on HAT culture medium [RPMI culture medium containing normal 10 % fetal calf serum supplemented with hypoxanthine (1 x 10⁻⁴ M), thymidine (1.5 x 10⁻³ M) and aminopterin (4 x 10⁻⁷ M)] and HT culture medium (HAT culture medium deprived of aminopterin) and the specific neutralizing clones were screened 10 - 14 days after the fusion. That is, positive clones were selected by the EIA method using the above-mentioned synthetic peptides as a solid phase, the fluorescent antibody method using H9/HTLV-IIIMN cells, or the Western blotting method using the HTLV-IIIMN purified viruses.

Using the culture supernatant or mouse ascites preparation of these positive clones, the neutralization test was conducted in the conventional manner. The neutralizing activity was determined by as to whether the syncytium formation induced by the infection of HTLV-IIIMN virus into MT4 cells was inhibited by the antibody or not.

In this way, there was obtained the hybridoma µ5.5 which produces the neutralizing antibody specific to the HTLV-IIIMN strain (Group I), said hybridoma having been deposited at the Fermentation Research Institute, Agency of Agricultural Science and Technology with the accession No. FERM BP-3402 on July 10, 1990 which was transferred from FERM P-11594 deposited on July 10, 1990.

As a result of the analysis of the characteristic feature of the monoclonal antibody µ5.5, it was found that said monoclonal antibody specifically binds to the exterial envelope glycoprotein gp120 of HTLV-IIIMN and is capable of completely inhibiting the cell-free viral infection at the antibody level of as low as 1 µg/ml. It was also found that this monoclonal antibody has a very strong neutralizing activity with the ability of inhibiting the cell to cell viral infection at the antibody level of 16 µg/ml. In addition, the experiment in Example 2 (see Tables 5 and 6) showed that the monoclonal antibody µ5.5 can effectively neutralize the other isolated viruses belonging to the same group (Group I).

The Group I-specific neutralizing monoclonal antibody has been established according to the above procedure. Using synthetic peptides belonging to the other Groups as the immunogen, the neutralizing monoclonal antibodies specific to Group II, III or IV could be established in the same manner. Accordingly, as shown in the experiment of Example 2 (cf. Tables 5, 6 and 7), it was confirmed that a mixture of neutralizing antibodies each belonging to either of all Groups [a mixture of five neutralizing antibodies each belonging to a different Group; e.g. a mixture of µ5.5 MoAb, 0.5β MoAb plus monoclonal antibodies each belonging to a different Group (either of Group II, III or IV)] can effectively prevent all types of the HIV-1 infections (also effective for the prevention of infection by clinical accident or infection between mother and child) or for the prevention of spreading of infection by HIV carriers.

The neutralizing monoclonal antibody used in the antibody preparation of the present invention is preferably a human chimeric neutralizing monoclonal antibody which is prepared by linking a gene fragment coding for the variable region of the mouse neutralizing monoclonal antibodies obtained hereinabove and a gene fragment coding for the constant region of human IgG and expressing the linked gene fragment, or a human reshaped neutralizing monoclonal antibody which is prepared by replacing only the complementarity determining regions (CDRs) of the variable region of human IgG with the CDRs from mouse neutralizing monoclonal antibodies, as described by Winter (GB patent publication No. 2,188,638, priority: March 27, 1986) since the antibody preparation comprising these chimeric antibodies can be advantageously used in the actual clinical situations with the lowest adverse side effects. The method for preparation of these human chimeric antibodies has been disclosed in detail in European patent publication No. 327000A.

## Claims

1. A peptide preparation which comprises at least three kinds of PND peptides of HIV gp120, wherein each of said PND peptides belongs a different Group selected from the following five groups (I) to (V);
(I) Group I: PND peptide having an amino acid sequence whose secondary structure is XXXBBBX,
(II) Group II: PND peptide having an amino acid sequence whose secondary structure is XXBBBBX,
(III) Group III: PND peptide having an amino acid sequence whose secondary structure is XXXBBBB,
(IV) Group IV: PND peptide having an amino acid sequence whose secondary structure is XXBBBBB, and
(V) Group V: PND peptide having an amino acid sequence whose secondary structure is XBBBBBX,
wherein B represents a β strand structure and X represents a turn structure or a coil structure,
said secondary structure sequence in each group being of the seven amino acids N-terminally adjacent to the GPGR region and estimated by the Robson's analytical method for secondary structure of protein (GOR method).

2. The peptide preparation of claim 1 wherein said PND peptides are five peptides and each peptide belongs to a different Group (either of Groups I to V).

3. An antibody preparation which comprises at least three specific neutralizing antibodies which recognize the PND peptide of HIV gp120, wherein each of said neutralizing antibodies belongs to a different Group selected from the following groups (I') to (V'):
(I') Group I': neutralizing antibody which specifically recognizes the PND peptide belonging to Group I in claim 1,
(II') Group II': neutralizing antibody which specifically recognizes the PND peptide belonging to Group II in claim 1,
(III') Group III': neutralizing antibody which specifically recognizes the PND peptide belonging to Group III in claim 1,
(IV') Group IV': neutralizing antibody which specifically recognizes the PND peptide belonging to Group IV in claim 1, and
(V') Group V': neutralizing antibody which specifically recognizes the PND peptide belonging to Group V in claim 1.

4. The antibody preparation of claim 3 wherein said neutralizing antibodies are five antibodies and each antibody belongs to a different Group (either of Groups I' to V').

5. The antibody preparation of claim 3 or 4 wherein at least one of said neutralizing antibodies is a monoclonal antibody.

6. The antibody preparation of claim 3 or 4 wherein all of said neutralizing antibodies are monoclonal antibodies.

7. The antibody preparation of claim 5 or 6 wherein said monoclonal antibody recognizes a superstructure (more than secondary structure) of the PND peptide.

8. A method for determining a type of neutralizing antibody against HIV, which comprises determining the type of the neutralizing antibody which recognizes the Group-specific qp120 antigen or PND peptide present in blood from an HIV-infected individual using the PND peptide preparation of any of claims 1 to 7, thereby determining as to which Group (Group I' to V') of neutralizing antibody in claim 3 said neutralizing antibody belongs to.

9. A method for determining a type of viral strain of HIV which comprises extracting an HIV DNA genome or a part thereof from an HIV viral strain to be tested, incorporating the HIV DNA into E. coli to clone the same, determining a nucleotide sequence of DNA coding for the PND region of HIV **env** gp120, and subjecting the primary amino acid sequence of the PND deduced from said nucleotide sequence to the Robson's analytical method for secondary structure of protein (GOR method), thereby determining as to which Group (Groups I to V) of PND peptide in claim 1 said HIV viral strain belongs to.

10. Use of the peptide preparation according to claim 1 or 2 for the preparation of a vaccine against HIV.

11. Use of the antibody preparation according to any one of claims 3 to 7 for the preparation of a HIV preventive or treating composition.

12. A process for the preparation of a peptide preparation according to claims 1 or 2 which comprises combining the respective PND peptides.

13. A process for the preparation of an antibody preparation according to any one of claims 3 to 7 which comprises combining the respective neutralizing antibodies.

## Patentansprüche

1. Peptidpräparation, umfassend mindestens drei Arten von PND-Peptiden von HIV gp120, wobei jedes der PND-Peptide zu einer unterschiedlichen Gruppe gehört, ausgewählt aus den folgenden fünf Gruppen (I) bis (V);
(I) Gruppe I: PND-Peptide mit einer Aminosäuresequenz, deren Sekundärstruktur XXXBBBX ist,
(II) Gruppe II: PND-Peptide mit einer Aminosäuresequenz, deren Sekundärstruktur XXBBBBX ist,
(III) Gruppe III: PND-Peptide mit einer Aminosäuresequenz, deren Sekundärstruktur XXXBBBB ist,
(IV) Gruppe IV: PND-Peptide mit einer Aminosäuresequenz, deren Sekundärstruktur XXBBBBB ist, und
(V) Gruppe V: PND-Peptide mit einer Aminosäuresequenz, deren Sekundärstruktur XBBBBBX ist,
wobei B eine β-Strangstruktur darstellt und X eine gedrehte Struktur oder eine Schraubenstruktur darstellt, wobei die Sekundärstruktursequenz jeder Gruppe aus den sieben Aminosäuren N-terminal benachbart zu der GPGR-Region besteht und bestimmt wird durch die analytische Methode nach Robson für die Sekundärstruktur von Proteinen (GOR-Methode).

2. Peptidpräparation nach Anspruch 1, wobei die PND-Peptide fünf Peptide sind und jedes Peptid zu einer unterschiedlichen Gruppe gehört (eine der Gruppen I bis V).

3. Antikörperpräparation, umfassend mindestens drei spezifische neutralisierende Antikörper, die das PND-Peptid von HIV gp120 erkennen, wobei jeder der neutralisierenden Antikörper zu einer unterschiedlichen Gruppe gehört, ausgewählt aus den folgenden Gruppen (I') bis V'):
(I') Gruppe I': Neutralisierender Antikörper, der spezifisch das PND-Peptid erkennt, das zur Gruppe I in Anspruch 1 gehört,
(II') Gruppe II': Neutralisierender Antikörper, der spezifisch das PND-Peptid erkennt, das zur Gruppe II in Anspruch 1 gehört,
(III') Gruppe III': Neutralisierender Antikörper, der spezifisch das PND-Peptid erkennt, das zur Gruppe III in Anspruch 1 gehört,
(IV') Gruppe IV': Neutralisierender Antikörper, der spezifisch das PND-Peptid erkennt, das zur Gruppe IV in Anspruch 1 gehört, und
(V') Gruppe V': Neutralisierender Antikörper, der spezifisch das PND-Peptid erkennt, das zur Gruppe V in Anspruch 1 gehört.

4. Antikörperpräparation nach Anspruch 3, wobei die neutralisierenden Antikörper fünf Antikörper sind und jeder Antikörper zu einer unterschiedlichen Gruppe gehört (einer der Gruppen I' bis V').

5. Antikörperpräparation nach Anspruch 3 oder 4, wobei mindestens einer der neutralisierenden Antikörper ein monoclonaler Antikörper ist.

6. Antikörperpräparation nach Anspruch 3 oder 4, wobei alle neutralisierenden Antikörper monoclonale Antikörper sind.

7. Antikörperpräparation nach Anspruch 5 oder 6, wobei die monoclonalen Antikörper eine Superstruktur (mehr als die Sekundärstruktur) des PND-Peptids erkennen.

8. Verfahren zur Bestimmung eines Typs eines neutralisierenden Antikörpers gegen HIV, umfassend die Bestimmung des Typs des neutralisierenden Antikörpers, der das gruppenspezifische gpl20-Antigen oder PND-Peptid erkennt, die in Blut von HIV-infizierten Individuen vorhanden sind, unter Verwendung der PND-Peptidpräparation nach einem der Ansprüche 1 bis 7, dadurch bestimmend, zu welcher Gruppe (Gruppe I' bis V') von neutralisierenden Antikörpern in Anspruch 3 der neutralisierende Antikörper gehört.

9. Verfahren zur Bestimmung eines Typs eines HIV-Virusstammes, umfassend die Gewinnung eines HIV-DNA-Genoms oder eines Teils davon aus einem zu testenden HIV-Virusstamm, Einbau der HIV-DNA in E. coli, um dieselbe zu clonieren, Bestimmung der Nucleotidsequenz der DNA, die die PND-Region von HIV env gp120 codiert, und Unterwerfen der primären Aminosäuresequenz des von der Nucleotidsequenz abgeleiteten PND der analytischen Methode nach Robson für die Sekundärstruktur von Proteinen (GOR-Methode), um zu bestimmen, zu welcher Gruppe (Gruppe I bis V) von PND-Peptiden in Anspruch 1 der HIV-Virusstamm gehört.

10. Verwendung der Peptidpräparation nach Anspruch 1 oder 2 für die Herstellung eines Impfstoffs gegen HIV.

11. Verwendung der Antikörperpräparation nach einem der Ansprüche 3 bis 7 für die Herstellung eines präventiven oder Behandlungsmittel für HIV.

12. Verfahren zur Herstellung einer Peptidpräparation nach Anspruch 1 oder 2, umfassend die Kombination der entsprechenden PND-Peptide.

13. Verfahren zur Herstellung einer Antikörperpräparation nach einem der Ansprüche 3 bis 7, umfassend die Kombination der entsprechenden neutralisierenden Antikörper.

## Revendications

1. Préparation peptidique qui comprend au moins trois types de peptides PND de gp120 de VIH, dans laquelle chacun desdits peptides PND appartient à un groupe différent choisi parmi les cinq groupes (I) à (V) suivants :
(I) groupe I : peptide PND ayant une séquence d'acides aminés dont la structure secondaire est XXXBBBX,
(II) groupe II : peptide PND ayant une séquence d'acides aminés dont la structure secondaire est XXBBBBX,
(III) groupe III : peptide PND ayant une séquence d'acides aminés dont la structure secondaire est XXXBBBB,
(IV) groupe IV : peptide PND ayant une séquence d'acides aminés dont la structure secondaire est XXBBBBB, et
(V) groupe V : peptide PND ayant une séquence d'acides aminés dont la structure secondaire est XBBBBBX,
où B représente une structure en brin β et X représente une structure en virage ou une structure en rouleau,
ladite séquence à structure secondaire dans chaque groupe étant des sept acides aminés adjacents en position N-terminale à la région GPGR et estimée par la méthode analytique de Robson pour la structure secondaire des protéines (méthode GOR).

2. Préparation peptidique selon la revendication 1, dans laquelle lesdits peptides PND sont cinq peptides et chaque peptide appartient à un groupe différent (l'un quelconque des groupes I à V).

3. Préparation d'anticorps qui comprend au moins trois anticorps neutralisants spécifiques qui reconnaissent le peptide PND de gp120 de VIH, dans laquelle chacun desdits anticorps neutralisants appartient à un groupe différent choisi pami les groupes (I') à (V') suivants :
(I') groupe I': anticorps neutralisant qui reconnaît spécifiquement le peptide PND appartenant au groupe I de la revendication 1,
(II') groupe II': anticorps neutralisant qui reconnaît spécifiquement le peptide PND appartenant au groupe II de la revendication 1,
(III') groupe III' : anticorps neutralisant qui reconnaît spécifiquement le peptide PND appartenant au groupe III de la revendication 1,
(IV') groupe IV' : anticorps neutralisant qui reconnaît spécifiquement le peptide PND appartenant au groupe IV de la revendication 1, et
(V') groupe V': anticorps neutralisant qui reconnaît spécifiquement le peptide appartenant au groupe V de la revendication 1.

4. Préparation d'anticorps selon la revendication 3, dans laquelle lesdits anticorps neutralisants sont cinq anticorps et chaque anticorps appartient à un groupe différent (l'un quelconque des groupes I' à V').

5. Préparation d'anticorps selon la revendication 3 ou 4, dans laquelle au moins l'un desdits anticorps neutralisants est un anticorps monoclonal.

6. Préparation d'anticorps selon la revendication 3 ou 4, dans laquelle tous lesdits anticorps neutralisants sont des anticorps monoclonaux.

7. Préparation d'anticorps selon la revendication 5 ou 6, dans laquelle ledit anticorps monoclonal reconnaît une superstructure (supérieure à une structure secondaire) du peptide PND.

8. Procédé pour déterminer un type d'anticorps neutralisant contre VIH, qui comprend la détermination du type de l'anticorps neutralisant qui reconnaît l'antigène gp120 ou le peptide PND spécifique de groupe présent dans le sang provenant d'un sujet infecté par VIH à l'aide de la préparation peptidique de peptides PND selon l'une quelconque des revendications 1 à 7, pour déterminer à quel groupe (groupes I' à V') d'anticorps neutralisant de la revendication 3 appartient ledit anticorps neutralisant.

9. Procédé pour déterminer un type de souche virale de VIH qui comprend l'extraction d'un ADN génomique de VIH ou d'une partie de celui-ci à partir d'une souche virale de VIH qui doit être testée, l'incorporation de l'ADN de VIH dans E. coli pour le cloner, la détermination d'une séquence nucléotidique d'ADN codant la région PND de env gp120 de VIH et la soumission de la séquence d'acides aminés primaire de PND déduite de ladite séquence nucléotidique à la méthode analytique de Robson pour la structure secondaire des protéines (méthode GOR) afin de déterminer à quel groupe (groupes I à V) de peptide PND de la revendication 1 appartient ladite souche virale de VIH.

10. Utilisation de la préparation peptidique selon la revendication 1 ou 2 pour la préparation d'un vaccin contre VIH.

11. Utilisation de la préparation d'anticorps selon l'une quelconque des revendications 3 à 7 pour la préparation d'une composition pour la prévention ou le traitement de VIH.

12. Procédé de préparation d'une préparation peptidique selon la revendication 1 ou 2 qui comprend la combinaison des peptides PND respectifs.

13. Procédé de préparation d'une préparation d'anticorps selon l'une quelconque des revendications 3 à 7 qui comprend la combinaison des anticorps neutralisants respectifs.
